# EUROPEAN PATENT SPECIFICATION

(11) **EP 4 525 734 B1**
(45) Date of publication and mention of the grant of the patent: **24.06.2026**
(21) Application number: 23727511.0
(22) Date of filing: 17.05.2023
(51) Int. Cl.: A61B 10/02, F16K 7/02

(54) **VALVE ASSEMBLY**
VENTILANORDNUNG
ENSEMBLE VANNE

(30) Priority: 18.05.2022 SE 2250596
(43) Date of publication of application: 26.03.2025
(73) Proprietor: Totle Development APS, 2640 Hedehusene (DK)
(72) Inventor: RISAGER, Flemming, 4700 Næstved (DK); JENSEN ROSENBERG, Per, 4684 Holmegaard (DK); VIDEBAEK, Karsten, 4040 Jyllinge (DK); ANDERSEN, Bjarne, 3600 Frederikssund (DK)
(74) Representative: Ström & Gulliksson AB
(86) International application number: PCT/EP2023/063223
(87) International publication number: WO 2023/222745

(56) References cited:
- WO-A1-2017/129735
- US-A- 2 314 767
- US-A- 4 730 635
- US-A- 5 826 621

## Description

### TECHNICAL FIELD

The present invention relates to a biopsy syringe comprising a valve assembly for regulating fluid flow in a channel.

### BACKGROUND

Biopsy, such as fine-needle aspiration biopsy (FNAB or FNA), fine-needle aspiration cytology (FNAC), or core needle biopsy (CNB), is a diagnostic procedure used to remove samples of tissue or fluid from an organ of the body or a lump found under the skin. Fine-needle aspiration may also be done to identify the type of cells inside a lump or to see how well treatment of an existing lump is working. FNA is commonly used to investigate lumps found in the breast or thyroid (a gland found in your neck) but it can also be used in other parts of the body. A core needle biopsy (CNB) is similar to an FNAB. A slightly larger, hollow needle is used to withdraw small cylinders (or cores) of tissue. A CNB is most often done in the health care provider's office with local anesthesia. The needle is inserted 3 to 6 times to obtain the samples, or cores. This is more time consuming than an FNAB, but it is more likely to give a clear result because more tissue is sampled for analysis. These methods are very useful ways of detecting cancer.

One biopsy technique used to evaluate breast tissue involves inserting a biopsy probe into the breast tissue region of interest to capture one or more tissue samples. Such a biopsy technique may utilize a vacuum to pull the tissue to be sampled into a sample notch of the biopsy probe, after which the tissue is severed and collected. A thin, hollow needle is inserted through the skin into the region of interest. Typically, there is a syringe attached to the needle.

WO2017/129735A1 discloses a convenient biopsy syringe for extracting fluid and/or tissue comprising a barrel with a proximal lumen and a distal lumen for collecting aspirated fluid/tissue. A plunger head of a plunger is sealingly engaged inside the barrel and a valve is located between the plunger head and a distal end of the syringe inside the barrel, separating the proximal lumen from the distal lumen. The valve regulates air flow between the distal lumen and the proximal lumen and is operated by a valve switch. Hence, the valve enables the biopsy syringe to attain two configurations; one open state where air may flow between the distal lumen and the proximal lumen and a closed state where air cannot flow there between.

However, even though the biopsy syringe disclosed in WO2017/129735A1 is an improvement over other existing solutions, the biopsy sample techniques are delicate. Any disruption in the air flow regulated by the valve assembly may damage the extracted samples, and therefore possibly cause misleading results during analysis. Furthermore, several valves available on the market are complex, resulting in cumbersome manufacturing processes. Hence, there is a need for an improved valve assembly for a biopsy syringe, being reliable and easy to produce.

Valve assemblies are known from e.g. US2314767 A, US4730635 A, and US5826621 A.

### SUMMARY

It is an object of the present invention, considering the disadvantages mentioned above, to provide a biopsy syringe according to claim 1.

This is advantageous since the valve assembly and its mechanisms are designed in such a way that the parts are forced to always deform in the same way. Hence, the valve assembly provides reproducibility for the switch between open and closed configurations, and the functioning of the valve assembly is thus very reliable. Other valve mechanisms in the art often rely on the movement of parts within the mechanism to establish a fluid flow path. Further, this valve assembly is easy to manufacture and can be applied in several different types of syringes in the art.

In one embodiment, when the valve assembly is in its open state, the tube is stretched transversally, perpendicularly to the extension of the channel.

In a further embodiment, when the valve assembly is in its open state, an opening is arranged between at least a part of the sphere and the tube.

In another embodiment, the tube has a platform extending laterally from the tube, wherein the platform is in communication with the switch and is configured to receive force applied to the tube by the switch.

The switch may comprise at least one leg in contact with said platform, which transfers a force applied to the switch to the platform.

In one embodiment, the channel is made of a material, which is more rigid than the material forming the tube.

In a further embodiment, the distal channel portion has a distal channel slit at its proximal end, and the proximal channel portion has a proximal channel slit at its distal end, wherein the distal and proximal slits are facing the sphere. The distal and proximal slits may extend transversally to the channel portions. Further, the distal and proximal slits may have a U-formed shape in the direction transversal to the distal and proximal channel portions.

In one embodiment, the valve assembly is arranged in the syringe. Preferably, the valve assembly is arranged in a distal end portion, between a distal tip and a barrel, of the syringe.

In another embodiment, the syringe comprises a platform receiving recess, configured to receive the platform when force is applied to the switch. Preferably the platform receiving recess is arranged in the distal end portion of the syringe.

The tube may be made of an elastomer, preferably being a rubber or a silicone. The biopsy syringe comprising such a valve assembly is advantageous since the use of biopsy syringes require accuracy and is a delicate process, and due to the advantages presented for the valve assembly above, a biopsy syringe comprising such a valve assembly is very beneficial. It is easy to produce and provides reliable results due to the reproducibility for the switch between open and closed configurations of the valve assembly.

In one embodiment, the biopsy syringe is a fine-needle aspiration syringe or a core-needle biopsy syringe.

Further features of the invention and its embodiments are set forth in the appended claims.

### BRIEF DESCRIPTION OF THE DRAWINGS

These and other aspects, features and advantages of which the invention is capable will be apparent and elucidated from the following description of non-limiting embodiments of the present invention, reference being made to the accompanying drawings, in which
Fig. 1 is a longitudinal cross-section of a biopsy syringe;
Fig. 2 is a longitudinal cross-section of a distal portion of the biopsy syringe;
Fig. 3 is a lateral cross-section of a distal portion of the biopsy syringe shown from a distal direction; and
Fig. 4 is a lateral cross-section of a distal portion of the biopsy syringe shown from a proximal direction.

### DETAILED DESCRIPTION OF THE EMBODIMENTS

Embodiments of the present invention will be described in more detail below with reference to the accompanying drawings in order for those skilled in the art to be able to carry out the invention. The invention may, however, be embodied in many different forms and should not be construed as limited to the embodiments set forth herein. Rather, these embodiments are provided so that this disclosure will be thorough and complete, and will fully convey the scope of the invention to those skilled in the art. The embodiments do not limit the invention, but the invention is only limited by the appended patent claims. Furthermore, the terminology used in the detailed description of the particular embodiments illustrated in the accompanying drawings is not intended to be limiting of the invention.

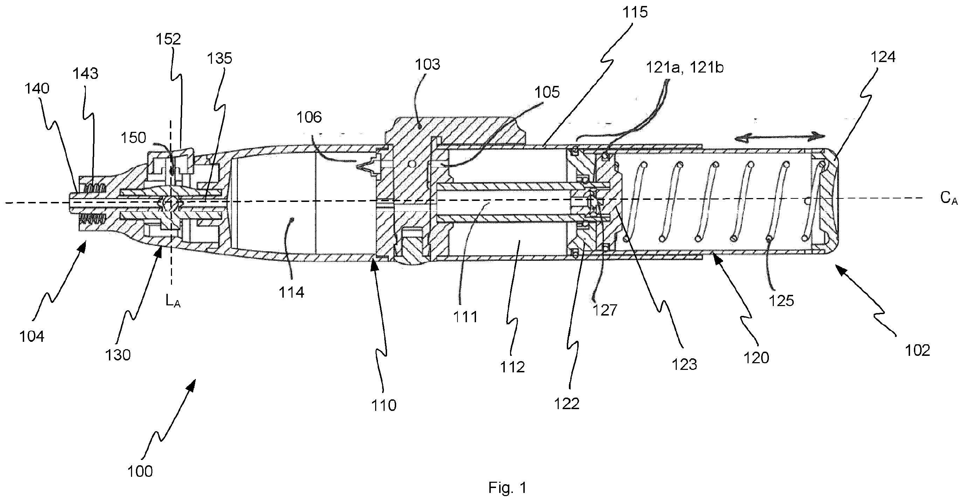

Embodiments of the present invention will now be described below with reference to Figs. 1 to 4.

Referring to Fig. 1, there is provided a biopsy syringe 100, extending along a proximodistal central axis C_{A}, and having a proximal end 102 and a distal end 104. Further, the syringe 100 has a lateral axis L_{A} extending perpendicularly in relation to the central axis C_{A}. The biopsy syringe 100 may also be referred to as a needle extraction syringe 100 herein, and may be a fine-needle aspiration syringe.

The biopsy syringe 100 comprises a hollow barrel 110 having a proximal lumen 112 and a distal lumen 114. A plunger 120 is arranged within the hollow barrel 110 and has a distal plunger stopper 122 and a proximal push element 124. The plunger stopper 122 is in fluid tight engagement with an internal surface wall 115 of the barrel 110, so that no liquids or gases may pass the barrier formed between the internal surface wall 115 and the plunger stopper 122.

When a distally directed force is applied onto the proximal push element 124, the plunger 120 is moved towards the distal end 104 of the biopsy syringe 100. Further, the plunger 120 comprises a spring member 125 providing the plunger 120 with resilient properties. The movement direction of the plunger 120 is indicated by the double ended arrow in Fig. 1.

On the proximal side of the plunger stopper 122 in Fig. 1, there is a sealing element 123. The plunger stopper 122 and the sealing element 123 are provided with check valves 121a, 121b. In addition, the plunger stopper 122 is equipped with an umbrella check valve 127, which in an open state is in fluid communication with an internal channel 111. The internal channel 111 extends along the central axis C_{A}. Further, the internal channel 111 extends through the proximal lumen 112.

The syringe 100 in Fig. 1 further comprises a pressure channel 105 between the distal lumen 114 with the proximal lumen 112. The pressure channel 105 extends through a pressure shift valve 103. At the distal end of the pressure channel 105, there is a deck bill check valve 106.

Further, the biopsy syringe 100 further comprises a distal end portion 130 and a distal tip 140, having a threaded internal portion 143 for connection of a needle or cannula (not shown) to the biopsy syringe 100. A channel 135 extends from the distal tip 140 to the barrel 110. Hence, the channel 135 provides communication between the distal tip 140 and the distal lumen 114 of the hollow barrel 110.

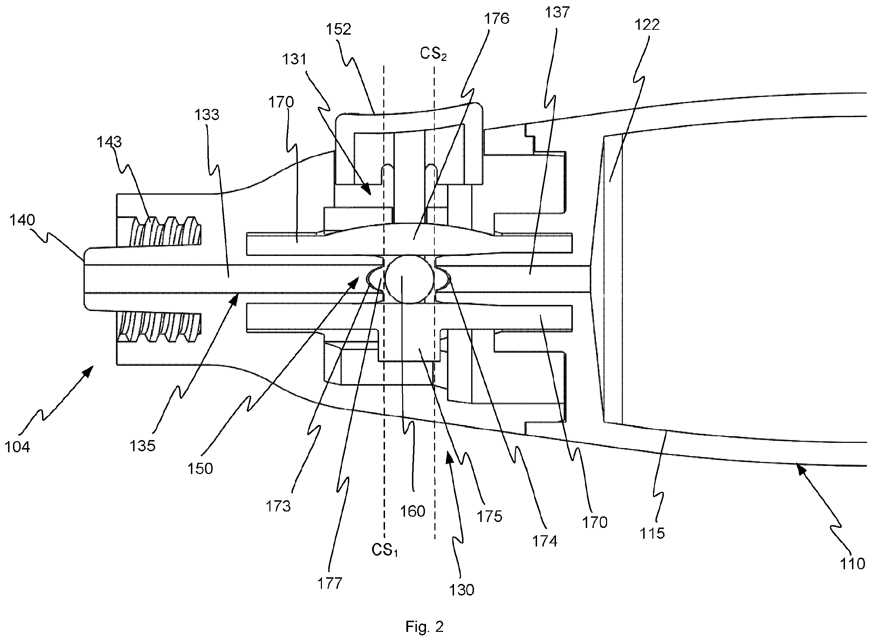

Moreover, as shown in Figs 1-4, the distal end portion 130 is provided with a valve assembly 150, comprising a switch 152. The valve assembly 150 is configurable between an open state and a closed state, and will be explained more in the following with reference to Figs. 2-4.

The biopsy syringe 100 disclosed in Fig. 1 is an exemplary biopsy syringe 100, and it should be appreciated that the valve assembly 150 may be comprised in other types of biopsy syringes, such as the biopsy syringe disclosed in WO2017/129735A1.

Preferably, the barrel 110, plunger 120, distal end portion 130, and distal tip 140 are made of a polymer material, such as polypropylene (PP).

The valve assembly 150 is a ball valve assembly, and is shown in more detail in Fig. 2 being an enlarged view of the distal end portion 130 of the syringe 100 shown in Fig. 1. The valve assembly 150 is configured to control a fluid and/or vacuum flow in the channel 135, which extends along the longitudinal proximodistal central axis C_{A}, said valve assembly 150 comprising the switch 152, a deformable tube 170 enclosing the channel 135, and the sphere 160 which is arranged within the tube 170 between a distal channel portion 133 and a proximal channel portion 137 of the channel 135. When the tube 170 is in a non-deformed resting state, the valve assembly 150 is in a closed state, and the sphere 160 is in direct contact with the tube 170, forming a fluid and/or vacuum seal with the tube 170. When a force is applied to the switch 152, the tube 170 is in a deformed non-resting state, and the valve assembly 150 is in an open state, whereby fluids are allowed pass the sphere 160.

As shown in Fig. 2, the valve assembly 150 comprises a sphere or ball 160 arranged within the tube 170 in a tube lumen 177. The sphere 160 is preferably formed from a polymer material, such as PP, or a metal, such as stainless steel. Preferably, the sphere 160 is formed from stainless steel.

The tube 170 is formed from a deformable, flexible material, and may for instance be formed from a polymer material, a silicone or a rubber. The tube 170 may be formed of an elastomer. Hence, the tube 170 is formed of a more flexible material than the channel 135. The channel 135 is made of a material more rigid than the tube 170. Therefore, the channel 135 can withstand a higher pressure or force exerted thereon than the tube 170.

When the tube 170 is submitted to strain, the material will deform, and flex back to its resting state when force is no longer applied and the tube 170 is no longer submitted to strain. In the valve assembly 150, the tube 170 is submitted to such strain through interaction with the switch 152.

With reference to Figs 2-4, the tube 170 is provided with a tube platform 175 and a longitudinal protrusion 176, arranged at opposite lateral sides of the tube 170. The tube platform 175 and/or the longitudinal protrusion 176 are preferably formed integrally with the tube 170. For instance, the tube 170 with the tube platform 175 and the longitudinal protrusion 176 is moulded in one piece.

An outer surface of the sphere 160 is in direct contact with an inner surface of the tube lumen 177, preventing fluids (such as liquids and gases) from entering the barrel 110. A diameter of the ball 160 is equal to or slightly greater than a diameter of the tube lumen 177. This dimensional relationship establishes a fluid seal between the inside surface of the tube lumen 177 and the sphere 160 when the tube 170 is not deformed (i.e. when the tube 170 is in its resting state). The fluid seal is a seal which prevents liquids and gas from passing the valve assembly 150. Hence, the valve assembly 150 may withhold a pressure difference (such as vacuum or close to vacuum)

The tube 170 and its tube lumen 177 encloses a part of the channel 135. The channel 135 has a distal channel portion 133 and a proximal channel portion 137. At its proximal end, the distal channel portion 133 is provided with a distal slit 173. The distal slit 173 extends transversally to the channel 135 and the distal channel portion 133. Correspondingly, the proximal channel portion 137 is provided with a proximal slit 174 at its distal end. The proximal slit 174 extends transversally to the channel 135 and the proximal channel portion 137. The channel 135 extends between and connects the distal tip 140 and the barrel 110, and is configured to receive the fluids aspirated or exhausted by the syringe 100. As shown in Fig. 2, the channel 135 is partly embedded within the tube 170.

The switch 152 is arranged in a distal end portion cavity 131 of the distal end portion 130, and projects laterally from the distal end portion 130 from an opening 134 in the distal end portion 130 of the syringe 100.

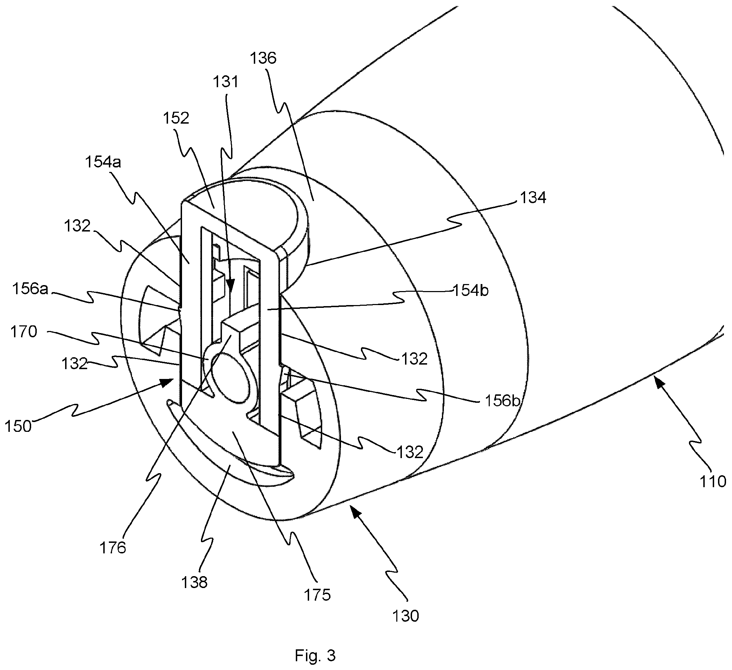

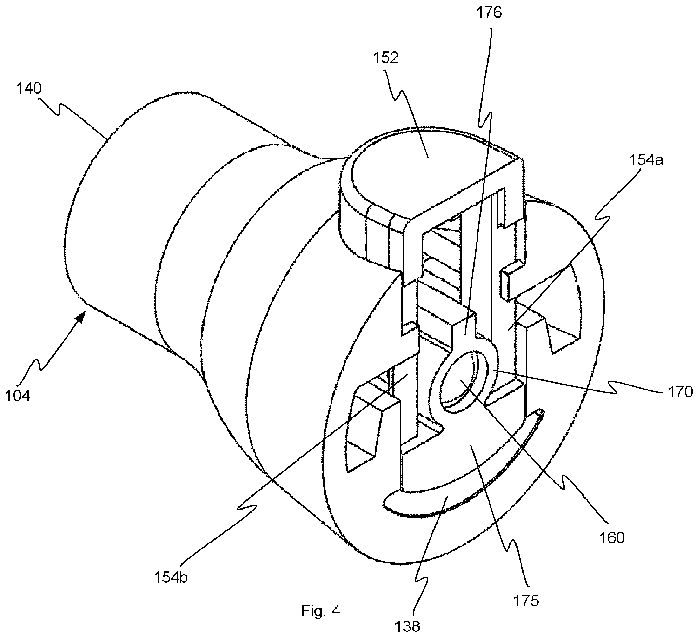

Fig. 3 is a cross-sectional view of the distal end portion 130 taken along the dashed line CS₁ in Fig. 2, while Fig. 4 is a cross-sectional view of the distal end portion 130 taken along the dashed line CS₂ in Fig. 2. As shown in Figs 3 and 4, the switch 152 comprises two switch legs 154a, 154b. As shown in Fig. 3, each leg 154a, 154b is provided with lateral leg projections 156a, 156b.

The distal end portion cavity 131 has internal surfaces 132 extending laterally, perpendicularly to the axis A, from the outer distal portion surface 136 towards the opposite side of the distal end portion 130. Opposite to the opening 134, the internal structure of the distal end portion cavity 131 defines a platform receiving recess 138.

### Switch open/closed state of valve assembly 150

With reference to Figs 2-4, the use of the ball valve assembly 150 will be explained more in detail. The valve assembly 150 allows for the switching between two different configurations; a first configuration being a closed state, where no fluids and/or vacuum may pass the valve 150, and a second configuration being an open state, in which fluids (such as liquid, tissues, gas or vacuum) may pass the valve 150 and enter or exit the barrel 110.

As described above, the valve assembly 150 is arranged in a distal end portion 130 of the needle extraction syringe 100. Hence, the valve assembly 150 is located between the distal tip 140 and the barrel 110, and controls the fluid/vacuum flow in the channel 135 there between. Fluids, such as liquids or tissue samples, are thus drawn into the syringe 100 by vacuum generated in the syringe 100. However, the valve assembly 150 may be arranged in another channel in another part of the syringe 100.

In the first configuration (see e.g. Fig. 3), the valve assembly 150 is in its closed state. The closed state is a resting state of the valve assembly 150. The switch legs 154a, 154b are in direct contact with the tube platform 175. No force is applied to the switch 152. Hence, the deformable tube 170 is not deformed or stretched. The sphere 160 forms a fluid and/or vacuum seal with the inner surface of the tube lumen 177 of the tube 170, preventing fluids from passing the valve assembly 150.

To bring the valve assembly 150 into its actuated open state, the user presses the switch 152 laterally towards the longitudinal central axis C_{A}. Hence, the force is directed along the lateral axis L_{A}. Thus, the switch 152 is actuated by a force applied along the lateral axis L_{A}. This causes the switch legs 154a, 154b to exert a laterally directed force on the tube platform 175. In turn, this causes the platform 175 to move laterally towards and into the platform receiving cavity 138. Hence, the open state of the valve assembly 150 is a deformed, non-resting state of the valve assembly 150. The switch 152 is arranged substantially perpendicular in relation to the tube 170, such that the switch 152 may exert a force directed along the lateral axis L_{A} onto the tube 170.

Since the platform 175 is formed in one piece with the tube 170, and since the tube 170 is made of a deformable material, the applied force and resulting movement of the platform 175 cause deformation of the tube 170. The channel 135 is not affected by the laterally directed force since it is formed from a more rigid material than the tube 170. As the tube 170 surrounds the channel 135, which is substantially unaffected by the user pushing the switch 152, a lateral stretching of the tube 170 is accomplished. The distal channel portion 133 and the proximal channel portion 137 thus act as force counter acting elements. Therefore, the pushing force exerted along the lateral axis L_{A} cause the tube 170 to be deformed in the area surrounding the tube lumen 177.

In this open, deformed, stretched state, the tube 170 attains a more elliptically shaped lateral cross-sectional in the tube lumen area 177. The shape of the sphere 160 residing within the tube lumen 177 is not affected by the applied force. Thus, the deformation results in that the fluid seal between the internal surface of the tube lumen 177 and the outer surface of the sphere 160 is lost, and at least one opening between the sphere 160 and the internal surface of the tube lumen is formed, and fluid may pass though the ball valve 150. The deformation of the tube 170 thus results in an opening arranged between the sphere 160 and the tube 170.

When the user removes the force applied to the switch 152, the tube 170 will spring back to its resting state and bring the valve assembly 150 back into its first configuration, i.e. its closed state.

Furthermore, as shown in Fig. 2, the distal and proximal slits 173, 174 have a concave, elliptical shape to prevent the ball 160 from blocking the channel 135. Consequently, if the sphere 160 comes into direct contact with one of the slits 173, 174 when the valve assembly 150 is in its open configuration, there would still be an opening for fluid flow around the sphere 160. The distal and proximal slits 173, 174 may have other shapes allowing for the passage of fluids when the sphere 160 comes into direct contact with the distal or proximal channel portion 133, 137 in the open state of the valve assembly 150, such as a tapered, or triangular shape.

Since the tube 170 is formed from an elastic material, the shape of the tube 170 will return to its initial shape once the force exerted on the switch 152 is released. Hence, the user may adjust the amount of gas/liquid the user wishes to allow to flow through the valve assembly 150 by selectively press the switch 152 more or less. This allows for easy adjustment of the valve assembly 150.

The lateral leg projections 156a, 156b hold the switch 152 in place by abutting the internal cavity surfaces 132, as shown in Fig. 3. When the platform 175 has been pressed as far into the platform receiving recess 138 as possible, the user cannot press the switch 152 further, and the valve assembly 150 has reached the open state having maximum flow capacity through the valve assembly 150.

Further, the valve assembly 150 disclosed herein is easy to produce and may be designed to fit various devices and is size adaptable. The dimensions of the valve assembly 150 are easily adjusted depending on need. More so, the valve assembly 150 is volume efficient, and thus possible to incorporate into small spaces while still being easy to manufacture.

### Accumulative generation of vacuum

The present disclosure further relates to accumulative generation of vacuum in the syringe 100 by pushing and releasing the plunger 120 repetitively. For the purpose, the syringe 100 preferably comprises the spring 125, which may be located between the sealing element 123 and the proximal push element 124 within the plunger 120, or any part in fixed connection with the proximal push element 124.

The possibility for accumulative generation of vacuum introduces several benefits for the user. One advantage is that it is possible to obtain a higher level of vacuum by removing air repetitively. A further advantage is that instead of one long movement of the plunger 120, additional smaller movements can generate the same, which allows a shorter design of the syringe 100. It may also be more preferable from an ergonomic perspective of the user to use several short movements instead of one long movement.

By pushing the plunger 120 towards the distal end 104 of the syringe 100 and releasing it towards the proximal end 102 of the syringe 100 repetitively, a vacuum is accumulated. The syringe 100 may either be open in the distal end 104, thereby aspirating additional tissue and/or fluid for every stroke (or accumulating the vacuum until the aspirating force is greater than a limit required for starting the process of drawing tissue into the distal lumen 114), or, if the syringe 100 is closed in the distal end 104, an accumulated vacuum may be generated inside the distal lumen 114, which is released when the distal end 104 is opened.

The syringe 100 disclosed herein further comprises the valve assembly 150 at the distal end 104 of the syringe 100 for maintaining and releasing a vacuum generated in the distal lumen 114 through the tip 140. In this way, a vacuum may be generated by one or several strokes while the valve assembly 150 is closed such that there is no connection between the distal lumen 114 and the exterior environment of the syringe 100 through the tip 140. When a suitable level of vacuum has been generated in the distal lumen 114, which may thereby serve as an accumulator tank in this configuration, the valve assembly 150 may be opened such that the distal lumen 114 is in fluid connection with the external environment of the syringe 100 (typically when a first hollow needle (not shown) is inserted to an area from which fluid and/or tissue is to be collected), thereby using the generated vacuum to aspirate the fluid and/or tissue into the distal lumen 114. Hence, fluid and/or tissue are drawn into the syringe 100 by the vacuum generated within the syringe 100.

In relation to the possibility to switch between the syringe 100 being closed and open in the distal end portion 130, the syringe 100 may be used in a number of scenarios involving "loading" the syringe 100, which is to be construed as generating a vacuum, which is not immediately released.

In one embodiment, the biopsy syringe 100 therefore has a loaded configuration, wherein the valve assembly 105 maintains the tip 140 in a closed position (in which the sphere 160 is blocking the channel 135) and a generated vacuum is held in the barrel 110, and a released configuration, wherein the valve assembly 150 maintains the tip 140 in an open position (where the switch 152 has been pressed and the tube 170 has been transversally deformed such that the sphere 160 at least partly is no longer in direct contact with the internal surface of the tube 170).

In one scenario, the syringe 100 may be loaded with a vacuum as described before a hollow needle is inserted into the area from which fluid and/or tissue is aspirated. This has the advantage that the generation of vacuum can be done without the risk of moving the first hollow needle inside the patient. Once the vacuum has been generated, i.e. the syringe 100 has been loaded, the needle can be carefully inserted, preferably without having to push or press any buttons. Once the first hollow needle has been positioned, the valve assembly 150 can be changed from closed to open (by pressing the switch 152 centrally), which causes the vacuum inside the barrel 110 to aspirate fluid and/or tissue into the distal lumen 114. The "loading" may include both a single stroke to generate vacuum or several strokes to accumulate a vacuum as described.

Alternatively, or in combination with this approach, the loading function can be used when the first hollow needle is already located in the area from which fluid and/or tissue is to be aspirated. In this example, the vacuum that is generated inside the barrel 110 can either be used immediately to aspirate tissue - in this case the valve assembly 150 maintains the tip 140 in an open position - or can be maintained in a closed position in order to accumulate a suitable level of vacuum, and then released. This procedure may be repeated.

### The use of the biopsy syringe 100

As mentioned above, the presently disclosed biopsy syringe 100 can create a vacuum inside the barrel 110 by pushing the plunger 120 towards the distal end 104 of the syringe 100. Optionally, the distal tip 140 is equipped with a needle (not shown) removably attached through the threaded portion 143.

To use the syringe 100, firstly, the syringe 100 is pumped a plurality of times, such as 10 times, by pressing the proximal push element 124 to generate vacuum. Secondly, a lesion of the patient is pierced with the needle. If necessary, an ultrasonic probe can be used to guide the needle. The switch 152 is pressed to open the valve assembly 150 such that tissue and/or liquid in the lesion is drawn into the syringe 100 by the generated vacuum. In an optional third step, the needle can be directed along a number of different tangents without existing the lesion while pressing a front end of an activation lever to obtain a biopsy sample. In this way, the lesion is "fanned". Fourthly, if more vacuum is needed, the syringe 100 can be pumped again without exiting the lesion. Then, steps three and four can be repeated until enough sample is obtained from. In a fifth step, the syringe 100 is extracted from the lesion and the syringe 100 is put in an ejection mode. Finally the user again pushes the proximal push element 124 to eject the sample onto e.g. a slide.

Consequently, one possible single-handed grip when using the syringe 100 is closing the index-, middle-, ring- and little fingers around the barrel 110 while the thumb is relatively loose. Typically, the little finger is placed on the distal part 104 of the syringe 100 towards the attached needle and the index finger on the upper part towards the proximal end 102. With such a grip, the thumb is typically stronger and more precisely moved downwards towards the palm of the user.

The principle of the presently disclosed biopsy syringe 100, allows for the creation of vacuum inside the barrel 110 by pushing the plunger 120 towards the distal end 104 of the syringe 100 instead of drawing it away from the distal end 104 towards the proximal end 102 of the syringe 100, as when using conventional syringes, involves the use of the plunger 120 having a plunger head 122 sealingly engaged inside the barrel 110 and enclosing the internal channel 111.

A closed volume on the proximal side of the plunger head 122, between the plunger head 122 and the sealing element 123, constitutes a vacuum chamber (not shown) having a variable volume, depending on how far distally the plunger 120 has been moved. The volume of the vacuum chamber is defined by the plunger head 122, the barrel 110 and the sealing element 123. When the proximal push element 124 is pressed, it pushes the plunger head 122 in the direction towards the distal end 104 of the syringe 100, and the vacuum chamber is mechanically expanded. Hence, when the vacuum chamber is expanded, the volume of the proximal lumen 112 decreases. In Fig. 1, the proximal lumen 112 is expanded and the vacuum chamber is thus not visible.

As described above, the use of biopsy syringes require accuracy and is a delicate process. The valve assembly 150 and its mechanisms are designed in such a way that the parts are forced to always deform in the same way. Hence, the valve assembly 150 provides reproducibility for the switch between open and closed configurations, and the functioning of the valve assembly 150 is thus reliable, which is of importance within this field. Other valve mechanisms in the art often rely on the movement of parts within the mechanism to establish a fluid flow path. However, several of these valves have parts with more than one degree of freedom, causing the risk of having configurational change which differ from one time to the other.

Vacuum in the present disclosure shall not be construed as absolute vacuum but a decreased pressure inside the syringe that allows tissue to be aspirated.

In the claims, the term "comprises/comprising" does not exclude the presence of other elements or steps. Furthermore, although individually listed, a plurality of means, elements or method steps may be implemented by e.g. a single unit or processor. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc. do not preclude a plurality. Reference signs in the claims are provided merely as a clarifying example and shall not be construed as limiting the scope of the claims in any way.

## Claims

1. A biopsy syringe extending along a longitudinal proximodistal central axis (C_{A}), wherein the biopsy syringe (100) comprises a valve assembly (150) for controlling fluid flow in a channel (135), which extends along the longitudinal proximodistal central axis (C_{A}), said valve assembly (150) comprising:
- a switch (152);
- a deformable tube (170) enclosing the channel (135); and
- a sphere (160) arranged within the tube (170) between a distal channel portion (133) and a proximal channel portion (137) of the channel (135);
wherein when the tube (170) is in a non-deformed resting state, the valve assembly (150) is in a closed state, and the sphere (160) is in direct contact with said tube (170), forming a fluid seal with the tube (170); and
wherein when a force is applied to the switch (152), the tube (170) is in a deformed non-resting state, and the valve assembly (150) is in an open state, whereby fluids are allowed pass the sphere (160).

2. The biopsy syringe according to claim 1, wherein when the valve assembly (150) is in its open state, the tube (170) is stretched transversally, perpendicularly to the extension of the channel (135).

3. The biopsy syringe according to claim 1 or 2, wherein when the valve assembly (150) is in its open state, an opening is arranged between at least a part of the sphere (160) and the tube (170).

4. The biopsy syringe according to any one of claims 1 to 3, wherein the tube (170) has a platform (175) extending laterally from the tube (170), wherein the platform (175) is in communication with the switch (152) and is configured to receive force applied to the tube (170) by the switch (152).

5. The biopsy syringe according to claim 4, wherein the switch (152) comprises at least one leg (154a, 154b) in contact with said platform (175), which transfers a force applied to the switch (152) to the platform (175).

6. The biopsy syringe according to any one of the preceding claims, wherein the channel (135) is made of a material which is more rigid than the material forming the tube (170).

7. The biopsy syringe according to any one of the preceding claims, wherein the distal channel portion (133) has a distal channel slit (173) at its proximal end, and the proximal channel portion (137) has a proximal channel slit (174) at its distal end, wherein the distal and proximal slits (173, 174) face the sphere (160).

8. The biopsy syringe according to claim 7, wherein the distal and proximal slits (173, 174) extend transversally to the channel portions (133, 137).

9. The biopsy syringe according to claim 7 or 8, wherein the distal and proximal slits (173, 174) have a U-formed shape in the direction transversal to the distal and proximal channel portions (133, 137).

10. The biopsy syringe according to any one of the preceding claims, wherein the valve assembly (150) is arranged in the syringe (100), preferably the valve assembly (150) being arranged in a distal end portion (130), between a distal tip (140) and a barrel (110), of the syringe (100).

11. The biopsy syringe according to claim 10, wherein the syringe (100) comprises a platform receiving recess (138), configured to receive the platform (175) when force is applied to the switch (152), preferably said platform receiving recess (138) being arranged in the distal end portion (130) of the syringe (100).

12. The biopsy syringe according to any one of the preceding claims, wherein the tube (170) is made of an elastomer, preferably being a rubber or a silicone.

13. The biopsy syringe according to any one of the preceding claims, wherein the biopsy syringe (100) is a fine-needle aspiration syringe or a core-needle biopsy syringe.

## Patentansprüche

1. Eine Biopsie-Spritze, die sich entlang einer longitudinalen proximodistalen Mittelachse (C_{A}) erstreckt, wobei die Biopsie-Spritze (100) eine Ventilanordnung (150) zum Steuern eines Fluidstroms in einem Kanal (135) aufweist, der sich entlang der longitudinalen proximodistalen Mittelachse (C_{A}) erstreckt, wobei die besagte Ventilanordnung (150) Folgendes aufweist:
- einen Schalter (152);
- einen verformbaren Schlauch (170), der den Kanal (135) umschließt; und
- eine Kugel (160), die innerhalb des Schlauchs (170) zwischen einem distalen Kanalabschnitt (133) und einem proximalen Kanalabschnitt (137) des Kanals (135) angeordnet ist;
wobei, wenn der Schlauch (170) in einem nicht-verformten Ruhezustand ist, die Ventilanordnung (150) in einem geschlossenen Zustand ist und die Kugel (160) in direktem Kontakt mit dem besagten Schlauch (170) ist und eine Fluiddichtung mit dem Schlauch (170) ausbildet; und
wobei, wenn eine Kraft auf den Schalter (152) ausgeübt wird, der Schlauch (170) in einem verformten Nicht-Ruhezustand ist und die Ventilanordnung (150) in einem offenen Zustand ist, wodurch Fluide die Kugel (160) passieren dürfen.

2. Die Biopsie-Spritze nach Anspruch 1,
wobei, wenn die Ventilanordnung (150) in ihrem offenen Zustand ist, der Schlauch (170) transversal, senkrecht zur Erstreckung des Kanals (135), gedehnt ist.

3. Die Biopsie-Spritze nach Anspruch 1 oder 2,
wobei, wenn die Ventilanordnung (150) in ihrem offenen Zustand ist, eine Öffnung zwischen wenigstens einem Teil der Kugel (160) und dem Schlauch (170) angeordnet ist.

4. Die Biopsie-Spritze nach einem der Ansprüche 1 bis 3,
wobei der Schlauch (170) eine Plattform (175) aufweist, die sich lateral von dem Schlauch (170) erstreckt, wobei die Plattform (175) in Verbindung mit dem Schalter (152) steht und eingerichtet ist zum Aufnehmen einer Kraft, die durch den Schalter (152) auf den Schlauch (170) ausgeübt wird.

5. Die Biopsie-Spritze nach Anspruch 4,
wobei der Schalter (152) wenigstens einen Schenkel (154a, 154b) in Kontakt mit der besagten Plattform (175) aufweist, der eine auf den Schalter (152) ausgeübte Kraft auf die Plattform (175) überträgt.

6. Die Biopsie-Spritze nach einem der vorhergehenden Ansprüche, wobei der Kanal (135) aus einem Material hergestellt ist, das steifer ist als das Material, das den Schlauch (170) ausbildet.

7. Die Biopsie-Spritze nach einem der vorhergehenden Ansprüche,
wobei der distale Kanalabschnitt (133) an seinem proximalen Ende einen distalen Kanalschlitz (173) aufweist und der proximale Kanalabschnitt (137) an seinem distalen Ende einen proximalen Kanalschlitz (174) aufweist,
wobei die distalen und proximalen Schlitze (173, 174) der Kugel (160) zugewandt sind.

8. Die Biopsie-Spritze nach Anspruch 7,
wobei die distalen und proximalen Schlitze (173, 174) sich transversal zu den Kanalabschnitten (133, 137) erstrecken.

9. Die Biopsie-Spritze nach Anspruch 7 oder 8,
wobei die distalen und proximalen Schlitze (173, 174) eine U-förmige Form in der Richtung transversal zu den distalen und proximalen Kanalabschnitten (133, 137) haben.

10. Die Biopsie-Spritze nach einem der vorhergehenden Ansprüche,
wobei die Ventilanordnung (150) in der Spritze (100) angeordnet ist, wobei die Ventilanordnung (150) vorzugsweise in einem distalen Endabschnitt (130), zwischen einer distalen Spitze (140) und einem Rohr (110), der Spritze (100) angeordnet ist.

11. Die Biopsie-Spritze nach Anspruch 10,
wobei die Spritze (100) eine Plattform-Aufnahmevertiefung (138) aufweist, die eingerichtet ist zum Aufnehmen der Plattform (175), wenn eine Kraft auf den Schalter (152) ausgeübt wird,
wobei die besagte Plattform-Aufnahmevertiefung (138) vorzugsweise in dem distalen Endabschnitt (130) der Spritze (100) angeordnet ist.

12. Die Biopsie-Spritze nach einem der vorhergehenden Ansprüche, wobei der Schlauch (170) aus einem Elastomer, vorzugsweise einem Kautschuk oder einem Silikon, hergestellt ist.

13. Die Biopsie-Spritze nach einem der vorhergehenden Ansprüche, wobei die Biopsie-Spritze (100) eine Feinnadel-Aspirationsspritze oder eine Kern-Nadel-Biopsie-Spritze ist.

## Revendications

1. Seringue de biopsie s'étendant le long d'un axe central longitudinal proximodistal (C_{A}), dans laquelle la seringue de biopsie (100) comprend un ensemble vanne (150) pour réguler un écoulement de fluide dans un canal (135), qui s'étend le long de l'axe central longitudinal proximodistal (C_{A}), ledit ensemble vanne (150) comprenant :
- un commutateur (152) ;
- un tube déformable (170) renfermant le canal (135) ; et
- une sphère (160) agencée à l'intérieur du tube (170) entre une partie de canal distale (133) et une partie de canal proximale (137) du canal (135) ;
dans laquelle, lorsque le tube (170) est dans un état au repos non déformé, l'ensemble vanne (150) est dans un état fermé, et la sphère (160) est en contact direct avec ledit tube (170), assurant l'étanchéité aux fluides vis-à-vis du tube (170) ; et
dans laquelle, lorsqu'une force est appliquée au commutateur (152), le tube (170) est dans un état non-au repos déformé, et l'ensemble vanne (150) est dans un état ouvert, selon laquelle les fluides peuvent passer la sphère (160).

2. Seringue de biopsie selon la revendication 1, dans laquelle lorsque l'ensemble vanne (150) est dans son état ouvert, le tube (170) est étiré transversalement, perpendiculairement à l'extension du canal (135).

3. Seringue de biopsie selon la revendication 1 ou 2, dans laquelle, lorsque l'ensemble vanne (150) est dans son état ouvert, une ouverture est agencée entre au moins une partie de la sphère (160) et le tube (170).

4. Seringue de biopsie selon l'une quelconque des revendications 1 à 3, dans laquelle le tube (170) présente une plateforme (175) s'étendant latéralement à partir du tube (170), dans laquelle la plateforme (175) est en communication avec le commutateur (152) et est configurée pour recevoir une force appliquée au tube (170) par le commutateur (152).

5. Seringue de biopsie selon la revendication 4, dans laquelle le commutateur (152) comprend au moins une patte (154a, 154b) en contact avec ladite plateforme (175), qui transfère à la plateforme (175) une force appliquée au commutateur (152).

6. Seringue de biopsie selon l'une quelconque des revendications précédentes, dans laquelle le canal (135) est composé d'un matériau qui est plus rigide que le matériau formant le tube (170).

7. Seringue de biopsie selon l'une quelconque des revendications précédentes, dans laquelle la partie de canal distale (133) présente une fente de canal distale (173) à son extrémité proximale, et la partie de canal proximale (137) présente une fente de canal proximale (174) à son extrémité distale, dans laquelle les fentes distale et proximale (173, 174) font face à la sphère (160) .

8. Seringue de biopsie selon la revendication 7, dans laquelle les fentes distale et proximale (173, 174) s'étendent transversalement aux parties de canal (133, 137).

9. Seringue de biopsie selon la revendication 7 ou 8, dans laquelle les fentes distale et proximale (173, 174) présentent une forme en U dans la direction transversale aux parties de canal distale et proximale (133, 137).

10. Seringue de biopsie selon l'une quelconque des revendications précédentes, dans laquelle l'ensemble vanne (150) est agencé dans la seringue (100), de préférence l'ensemble vanne (150) étant agencé dans une partie d'extrémité distale (130), entre une pointe distale (140) et un cylindre (110), de la seringue (100).

11. Seringue de biopsie selon la revendication 10, dans laquelle la seringue (100) comprend un évidement (138) de réception de plateforme, configuré pour recevoir la plateforme (175) lorsque la force est appliquée au commutateur (152), de préférence ledit évidement (138) de réception de plateforme étant agencé dans la partie d'extrémité distale (130) de la seringue (100).

12. Seringue de biopsie selon l'une quelconque des revendications précédentes, dans laquelle le tube (170) est composé d'un élastomère, de préférence un caoutchouc ou un silicone.

13. Seringue de biopsie selon l'une quelconque des revendications précédentes, dans laquelle la seringue de biopsie (100) est une seringue d'aspiration à aiguille fine ou une seringue de biopsie à aiguille centrale.
